# EUROPEAN PATENT APPLICATION

(11) **EP 2 476 459 A1**
(43) Date of publication of application: **18.07.2012**
(21) Application number: 09849264.8
(22) Date of filing: 15.09.2009
(51) Int. Cl.: A61N 5/06, C04B 35/10, C04B 35/14, A61F 7/00

(54) **PORTABLE DEVICE FOR TREATING ATOPY**

(30) Priority: 08.09.2009 KR 20090084650
(71) Applicant: Lim, Dong Gi, Uijeongbu-si, Gyeonggi-do 480-101 (KR)
(72) Inventor: Lim, Dong Gi, Uijeongbu-si, Gyeonggi-do 480-101 (KR)
(74) Representative: Lambert, Ian Robert
(86) International application number: PCT/KR2009/005243
(87) International publication number: WO 2011/030947

(57) **Abstract**

Disclosed is a portable atopy treatment device which can treat atopic pruritus and the like based on far infrared light emission. The atopy treatment device includes a body provided at a front side thereof with a recess; a ceramic heater mounted on the recess of the body and formed of a ceramic composite comprising alumina (Al₂O₃), barium oxide (BaO), silica (SiO₂) and neodymium oxide (Nd₂O₃), the ceramic heater being provided therein with a resistive heating element to emit far infrared light while being indirectly heated by heat from the resistive heating element; a power supply unit placed either inside the body or detachably outside the body and connected to an external power source to supply electric current to the resistive heating element; and a gripper extending from the body.

## Description

### [Technical Field]

The present invention relates to an atopy treatment device configured to treat a disease using heat, and more particularly, to a portable device for atopy treatment, which is portable, emits far infrared light at wavelengths ranging from 1,000 to 25,000 nm, and may treat not only pruritus caused by atopy, but also pimples, dermatophytosis, eczema, and the like, which cause the pruritus, using emitted heat and far infrared light.

### [Background Art]

Recently, a variety of ceramic thermotherapy devices have been produced. Among thermotherapy devices, a far infrared treatment device has attracted the most attention.

In general, far infrared light has a long wavelength ranging from 1,000 to 25,000 nm. Since far infrared light has such a long wavelength, it is very absorbent and permeates deep into the skin. Such thermal reaction of far infrared light is conducive to removal of disease causing bacteria.

The far infrared treatment device is configured to facilitate use of far infrared rays in order to use such characteristics of the far infrared rays in healing a person's body, and includes an alumina-based ceramic heater, which includes 90 percent by weight (wt%) or more of alumina (Al₂O₃).

However, the alumina-based ceramic heater containing 90 wt% or more of alumina emits far infrared light having wavelengths in the range of 10,000 to 20,000 nm.

### [Disclosure]

### [Technical Problem]

The present invention is directed to providing a portable device for atopy treatment, which can replace a conventional alumina-based ceramic heater, can reduce the amount of alumina in a ceramic heater, and includes predetermined amounts of silica, barium oxide and neodymium oxide, thereby enabling emission of far infrared light in a wide wavelength range of 1,000 to 25,000 nm.

### [Technical Solution]

In accordance with one aspect of the present invention, a portable device for atopy treatment includes: a body provided at a front side thereof with a recess; a ceramic heater mounted on the recess of the body and formed of a ceramic composite comprising alumina (Al₂O₃), barium oxide (BaO), silica (SiO₂) and neodymium oxide (Nd₂O₃), the ceramic heater being provided therein with a resistive heating element to emit far infrared light while being indirectly heated by heat from the resistive heating element; a power supply unit placed either inside the body or detachably outside the body and connected to an external power source to supply electric current to the resistive heating element; and a gripper extending from the body.

The ceramic heater may include 40 to 50 wt% of alumina, 20 to 35 wt% of barium oxide, 10 to 15 wt% of silica, 5 to 15 wt% of neodymium oxide, and 2 to 5 wt% of at least one of germanium oxide (Ge₂O₃), titanium oxide (TiO₂), magnesium oxide (MgO), calcium oxide (CaO), and iron oxide (Fe₂O₃).

### [Advantageous Effects]

In accordance with the present invention, the portable device for atopy treatment includes a gripper configured to provide portability and a safety net configured to provide safety.

Further, in accordance with the present invention, the portable device for atopy treatment may uniformly emit far infrared light in a wider wavelength range of 1,000 to 25,000 nm than that of the conventional alumina-based ceramic heater, thereby providing effects of treating atopy causing dermatitis pruritus together with pimples, dermatophytosis, eczema, and the like, which cause pruritus.

### [Description of Drawings]

Fig. 1 is a perspective view of a portable device for atopy treatment according to one exemplary embodiment of the present invention.
Fig. 2 is a perspective view of a portable device for atopy treatment according to another exemplary embodiment of the present invention, to which a power supply unit is detachably attached.
Fig. 3 is a side-sectional view of the portable device in which a ceramic heater is received in a body of the portable device.
Fig. 4 illustrates one embodiment of a ceramic heater applied to the present invention.
Fig. 5 is a graph depicting visual analogue scale (VAS) values of an experimental group and a control group over time.

### [Best Mode]

The above and other aspects, features, and advantages of the invention will become apparent from the following detailed description of exemplary embodiments in conjunction with the accompanying drawings. It should be understood that the present invention is not limited to the following embodiments and may be embodied in different ways, and that the embodiments are provided to provide complete disclosure of the invention and a thorough understanding of the invention to those skilled in the art. The scope of the invention is limited by the accompanying claims and equivalents thereof. Like elements will be indicated by like reference numerals throughout the specification.

Exemplary embodiments of the present invention will be described in detail with reference to the accompanying drawings.

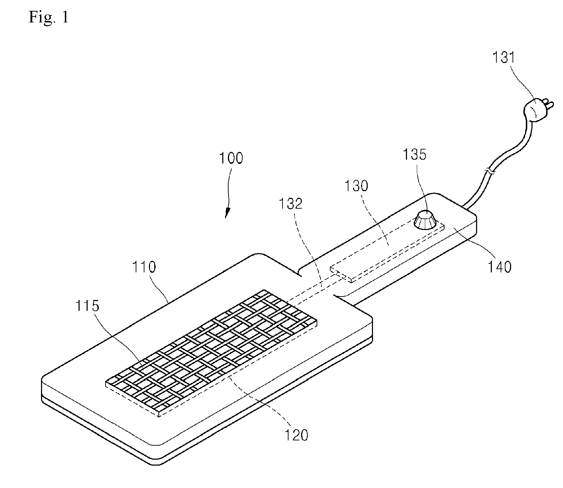

Fig. 1 is a perspective view of a portable device for atopy treatment according to one exemplary embodiment of the present invention.

Referring to Fig. 1, the portable device 100 for treating atopy includes a body 110, a ceramic heater 120, a power supply unit 130, and a gripper 140.

The body 110 constitutes an outer appearance of the device 100 and is provided at a front side thereof with a recess on which the ceramic heater 120 is mounted. The body 110 may be made of stainless steel or aluminum, which exhibits excellent heat reflecting characteristics.

The ceramic heater 120 is made of a ceramic composite mainly composed of alumina (Al₂O₃), and is mounted on the recess within the body.

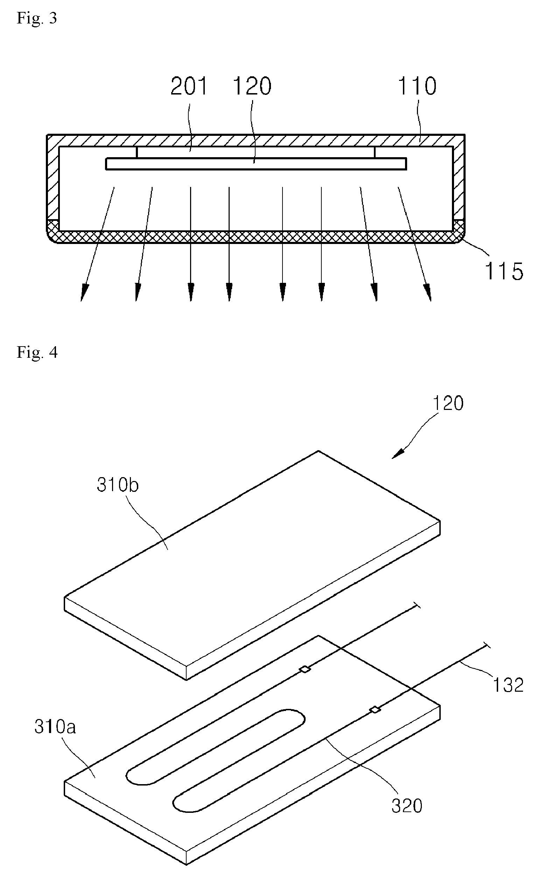

Fig. 3 is a side-sectional view of the portable device in which a ceramic heater is received in a body of the portable device.

Referring to Fig. 3, the ceramic heater 120 is mounted on the recess within the body 110. Here, the ceramic heater 120 may be mounted thereon via a coupling member 201 such as adhesives or the like. Alternatively, the ceramic heater 120 may be mounted on the recess within the body 110 by machining the recess or the rim of the ceramic heater 120 and fitting the ceramic heater to the recess without using the coupling member 201.

The ceramic heater 120 is provided therein with a resistive heating element 320 (see Fig. 4). The resistive heating element generates heat upon application of electric current thereto, and may be composed of tungsten (W), molybdenum (Mo), rhenium (Re), platinum (Pt), nickel (Ni), chromium (Cr), and the like.

The ceramic heater 120 is indirectly heated by heat generated from the resistive heating element. The temperature of the resistive heating element may vary in the range of 300 to 1800°C depending on electric current supplied from the power supply unit. Similarly, the surface temperature of the ceramic heater 120 indirectly heated by the resistive heating element may vary in the range of 300 to 1000°C depending on electric current supplied from the power supply unit.

As the ceramic heater 120 is heated, it emits far infrared light in the wavelength range of 1,000 to 25,000 nm. Far infrared light can be divided into first far infrared light in the wavelength range of 1,000 to 3,000 nm, second far infrared light in the wavelength range of 3,000 to 6,000 nm, and third far infrared light in the wavelength range of 6,000 to 25,000 nm.

The far infrared light in the wavelength range of 1,000 to 25,000 nm include all components of the first far infrared light, the second far infrared light, and the third far infrared light. Thus, the device 110 according to the present invention may emit all of the first, second and third far infrared light. This is because the ceramic heater 120 is made of the ceramic composite mainly composed of alumina.

Specifically, the ceramic heater 120 may include 40 to 50 wt% of alumina, 20 to 35 wt% of barium oxide, 10 to 15 wt% of silica, 5 to 15 wt% of neodymium oxide, and 2 to 5 wt% of at least one of germanium oxide (Ge₂O₃), titanium oxide (TiO₂), magnesium oxide (MgO), calcium oxide (CaO), and iron oxide (Fe₂O₃).

For Examples 1 and 2 of the ceramic heaters satisfying this composition, and Comparative Examples 1 and 2 of the conventional alumina-based ceramic heater dissatisfying the composition, emissivity of the first, second and third far infrared light was measured with reference to an emissivity of 1.000, which a black body has according to the wavelength ranges, using an FT-IR spectrometer (Shimadzu Co., Ltd., Japan) under the condition that each of the ceramic heaters has a surface temperature of 500°C.

Here, the ceramic heater employed in Example 1 was composed of 45 wt% of alumina, 30 wt% of barium oxide, 12.5 wt% of silica, 10 wt% of neodymium oxide (Nd₂O₃), 1.5 wt% of germanium oxide, and 1 wt% of magnesium oxide. As a result of measurement, each emissivity of the first, second and third far infrared light was 0.8 or more at the ceramic heater's surface temperature of 500°C.

Also, the ceramic heater of Example 2 was composed of 40 wt% of alumina, 35 wt% of barium oxide, 10 wt% of silica, 12.5 wt% of neodymium oxide, 1.5 wt% of germanium oxide, and 1 wt% of magnesium oxide. As a result of measurement, as in Example 1, each emissivity of the first, second and third far infrared light was 0.8 or more at the ceramic heater's surface temperature of 500°C.

On the other hand, for the ceramic heater of Comparative Example 1, which was composed of 90 wt% of alumina, 5 wt% of silica, 2.5 wt% of germanium oxide, and 2.5 wt% of magnesium oxide, the emissivity of the third far infrared light was 0.8 or more at the ceramic heater's surface temperature of 500 °C , but each emissivity of the first and second far infrared light was 0.8 or less.

For the ceramic heater of Comparative Example 2, which was composed of 50 wt% of alumina, 45 wt% of silica, 2.5 wt% of germanium oxide, and 2.5 wt% of magnesium oxide, each emissivity of the first and second far infrared light was 0.8 or more at the ceramic heater's surface temperature of 500 °C, but the emissivity of the third far infrared light was equal to or lower than 0.8.

Consequently, it can be seen that the conventional alumina-based ceramic heater has good emissivity of the third far infrared light, but relatively low emissivity of the first and second far infrared light. Also, if the amount of silica is greater than 40 wt%, it can be seen that the emissivity of the third far infrared light is significantly lowered. On the other hand, for the ceramic heaters according to the present invention, it can be seen that all of the first, second and third far infrared light have good emissivity.

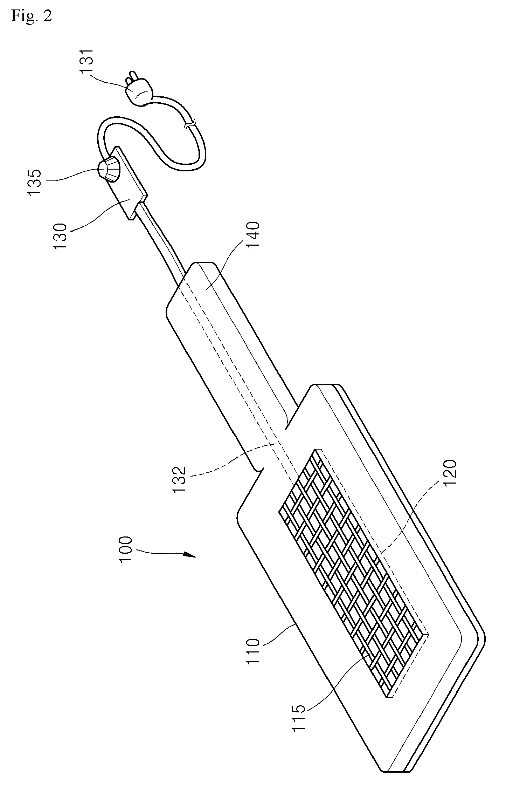

Next, the power supply unit 130 is connected to an external power source and supplies electric current to allow the resistive heating element to generate heat. Specifically, the power supply unit 130 includes a current control circuit (not shown) and is embedded within the body 110 or the gripper 140. The power supply unit 130 is connected to the external power source through a plug 131 and supplies electric current to the resistive heating element through an electric-current supply line 132. The power supply unit 130 may be detachably provided outside the body 110, as shown in Fig. 2.

The gripper 140 extends from the body to be easily gripped by a user when the atopy treatment device according to the embodiment is used to treat atopy. The gripper 140 may be integrally formed with the body 110, or may be separately manufactured from the body 110 to be coupled to the body 110 by fitting or the like.

Further, the gripper 140 may be further provided at the front, back or lateral side thereof with the current control switch 135 to control the amount of electric current supplied to the resistive heating element. The current control switch 135 has a knob shape, as shown in Fig. 1, or a press switch shape, and is connected to the power supply unit 130.

If the current control switch 135 is further provided to the gripper 140, it is possible to control the amount of heat emitted from the resistive heating element by controlling on/off and the amount of electric current supplied to the resistive heating element through the current control switch 135. Accordingly, it is also possible to control the temperature of the ceramic heater 120 through the resistive heating element. Thus, it is possible to set up the temperature of the ceramic heater as desired by a user.

Meanwhile, the body 110 may further include a safety net 115 which covers the front side of the ceramic heater 120 so as to prevent burns or the like due to separation of the ceramic heater from the recess of the body 110 or due to a user's mistake. The safety net 115 may be made of stainless steel or aluminum through which heat can be emitted.

Further, an insulator (not shown) may be disposed on parts of the ceramic heater mounted inside the body 110, except for the front side thereof, that is, to surround the back and lateral sides of the ceramic heater 120.

The power supply unit 130, the gripper 140, and the like may be damaged by heat generated from the ceramic heater 120. To prevent the power supply unit 130 and the gripper 140 from being damaged due to heat, the insulator may be disposed in the parts of the ceramic heater 120 except for the front side thereof. In this case, heat generated from the ceramic heater 120 is shielded by the insulator and prevented from being supplied to the power supply unit 130 or the gripper 140.

In addition, when the insulator is provided, it is possible to concentrate heat upon the front side of the ceramic heater.

Fig. 4 illustrates one embodiment of the ceramic heater applied to the present invention.

Referring to Fig. 4, the ceramic heater 120 may be embodied by a stack of two ceramic plates 310a, 310b with the resistive heating element 320 interposed between facing surfaces thereof.

Obviously, three or more ceramic plates may be stacked to constitute the ceramic heater. In this case, the resistive heating element 320 may be disposed on each of the facing surfaces, or may be disposed only on one or part of the facing surfaces.

Effectiveness of the atopy treatment device including the ceramic heater of Example 1 in healing atopy pruritus was tested in relation with surface temperature. For the test, the treatment device for an experimental group was adjusted such that the ceramic heater had a surface temperature of 500 °C, and the treatment device for a control group was adjusted such that the ceramic heater had a surface temperature of 25°C.

This test was applied to 84 subjects, 42 subjects of which were tested using the atopy treatment device with the ceramic heater under the condition for the experimental group and the 42 other subjects were tested using the atopy treatment device with the ceramic heater under the condition for the control group.

### 1. Degree of improvement from atopy pruritus

Null hypothesis: µₜ - µ_{c} ≤ δ, alternative hypothesis: µₜ - µ_{c} > δ
(where µₜ is the degree of improvement in the experimental group, µ_{c} is the degree of improvement in the control group, and δ is 0 or 0.3)

The degree of improvement from atopy pruritus: (VAS before treatment - VAS after two weeks)÷(VAS before treatment) × 100

Here, the VAS is a pruritus pain value represented by 0 ∼ 10, which can be generally classified as follows.
0: no itchiness
1~3: slight itchiness
4-6: medium itchiness
7~8: severe itchiness
9~10: daily life is hindered due to very severe pruritus

If the null hypothesis is accepted, it means that there is substantially no difference between the degree of improvement from the pruritus in the experimental group and the degree of improvement from the pruritus in the control group. On the other hand, if the alternative hypothesis is accepted, it means that the degree of improvement from the pruritus in the experimental group is higher than that of the control group.

Table 1 shows difference in the degree of improvement from atopy pruritus between the experimental group and the control group

**[Table 1]**

| | Subjects | Degree of improvement (average) | Difference in degree (µₜ - µ_{c}) |
|---|---|---|---|
| Experimental group | 42 | 0.459 (µₜ) | 0.306 |
| Control group | 42 | 0.153 (µ_{c}) | |

Referring to Table 1, the degree of improvement (µₜ) from atopy pruritus in the experimental group showed an average of 0.469, and the degree of improvement (µ_{c}) from atopy pruritus in the control group showed an average of 0.153. The difference (µₜ - µ_{c}) in the degree of improvement from atopy pruritus between the experimental group and the control group is 0.306, so that the null hypothesis cannot be accepted when δ is either 0 or 0.3 Accordingly, the alternative hypothesis is accepted and it is thus regarded that the degree of improvement from atopy pruritus of the experimental group is definitely higher than that of the control group.

### 2. Degree of reduction in VAS

Null hypothesis: µₜ - µ_{c} ≤ δ, alternative hypothesis: µₜ - µ_{c} > δ
(where µₜ is an average degree of reduction in VAS of the experimental group, and µ_{c} is an average degree of reduction in VAS of the control group)
VASC: VAS before using the treatment device - VAS for 14 days
(where VAS is a pruritus pain value)

If the null hypothesis is accepted, it means that the degree of reduction in VAS of the experimental group is smaller than that of the control group. On the other hand, if the alternative hypothesis is accepted, it means that the degree of reduction in VAS of the experimental group is higher than that of the control group.

Table 2 shows difference in the degree of reduction in VAS between the experimental group and the control group

**[Table 2]**

| | Subjects | Degree of reduction in VAS (average) | Difference in degree of reduction in VAS (µₜ - µ_{c}) |
|---|---|---|---|
| Experimental group | 42 | 3.458 (µₜ) | 2.062 |
| Control group | 42 | 1.396 (µ_{c}) | |

Referring to Table 2, the average VAS of the experimental group before using the test device was 6.237, and the average VAS of the experimental group 14 days after use of the treatment device was 2.779, showing that an average degree of reduction (µₜ) in VAS of the experimental group was 3.458. On the other hand, the average VAS of the control group before using the test device was 6.354, and an average VAS of the control group 14 days after use of the treatment device was 4.958, showing that an average degree of reduction (µ_{c}) in VAS of the control group was 1.396. The difference (µₜ - µ_{c}) in the degree of reduction in VAS between the experimental group and the control group 14 days after use of the treatment device was 2.062, and δ > 0, whereby the alternative hypothesis can be accepted instead of the null hypothesis. Thus, it is regarded that the degree of reduction in VAS of the experimental group is definitely greater than that of the control group, which means that pruritus due to atopy is effectively relieved in the experimental group.

### 3. Extent of pruritus over time

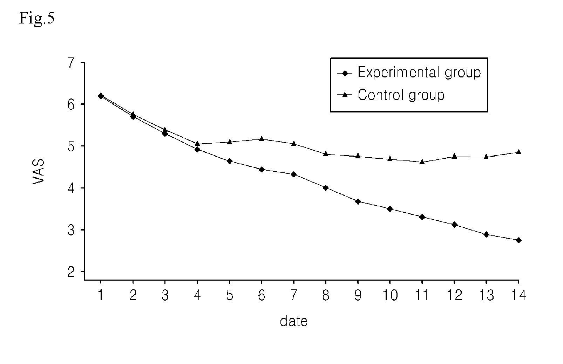

Fig. 5 shows change in VAS values of the experimental group and the control group 14 days after use of the treatment device.

Referring to Fig. 4, for 3 days after use of the treatment device, the experimental group and the control group were similar with regard to the degree of reduction in VAS. 4 days after use of the treatment device, the VAS of the experimental group was continuously decreased, but the VAS of the control group was not decreased any more.

This result means that there was an effect of decreasing VAS continuously as well as in the early stage when the ceramic heater for the experimental group was used, whereas there was little effect of decreasing VAS only in the early stage when the ceramic heater for the control group was used.

As described above, the portable device for atopy treatment according to the embodiment may emit far infrared light in a wider wavelength range than the conventional alumina-based ceramic heater, thereby providing improvement in healing pruritus.

Accordingly, there are significant effects in healing atopy pruritus together with pimples, dermatophytosis, eczema, etc., which cause the pruritus.

Although the present invention has been described with reference to some exemplary embodiments in conjunction with the drawings, it should be understood that various modifications, variations, and alterations can be made by a person having ordinary knowledge in the art without departing from the spirit and scope of the invention. Therefore, the scope of the invention should be limited only by the accompanying claims and equivalents thereof.

## Claims

1. A portable device for atopy treatment comprising:
a body provided at a front side thereof with a recess;
a ceramic heater mounted on the recess of the body and formed of a ceramic composite comprising alumina (Al₂O₃), barium oxide (BaO), silica (SiO₂) and neodymium oxide (Nd₂O₃), the ceramic heater being provided therein with a resistive heating element to emit far infrared light while being indirectly heated by heat from the resistive heating element;
a power supply unit placed either inside the body or detachably outside the body and connected to an external power source to supply electric current to the resistive heating element; and
a gripper extending from the body.

2. The portable device of claim 1, wherein the resistive heating element is selected among tungsten (W), molybdenum (Mo), rhenium (Re), platinum (Pt), nickel (Ni), and chromium (Cr).

3. The portable device of claim 1, wherein the ceramic heater comprises 40 to 50 wt% of alumina, 20 to 35 wt% of barium oxide, 10 to 15 wt% of silica, 5 to 15 wt% of neodymium oxide, and 2 to 5 wt% of at least one of germanium oxide (Ge₂O₃), titanium oxide (TiO₂), magnesium oxide (MgO), calcium oxide (CaO), and iron oxide (Fe₂O₃).

4. The portable device of claim 1, wherein the body further comprises a safety net covering a front side of the ceramic heater.

5. The portable device of claim 1, wherein the body further comprises an insulator disposed to surround back and lateral sides of the ceramic heater.

6. The portable device of claim 1, further comprising:
a current control switch disposed on a front or back side of the gripper and controlling the electric current supplied to the resistive heating element.

7. The portable device of claim 1, wherein the ceramic heater is a stack of multiple ceramic plates, with resistive heating elements disposed on facing surfaces thereof.

8. The portable device of claim 1, wherein the ceramic heater has a surface temperature varying within the range of 300 to 1000 °C according to the electric current supplied from the power supply unit.
